(19) Europäisches Patentamt
European Patent Office
Office européen des brevets

(11) Veröffentlichungsnummer: **0 129 170**
**B1**

# (12) EUROPÄISCHE PATENTSCHRIFT

(45) Veröffentlichungstag der Patentschrift: **10.01.90**

(51) Int. Cl.⁴: **C 07 D 249/08**, A 01 N 43/64

(21) Anmeldenummer: **84106595.6**

(22) Anmeldetag: **08.06.84**

(54) **Triazolyl-ketonoxime und -dioxime, Verfahren zu ihrer Herstellung und ihre Verwendung als Pflanzenwachstumsregulatoren.**

(30) Priorität: **11.06.83 DE 3321183**

(43) Veröffentlichungstag der Anmeldung: **27.12.84 Patentblatt 84/52**

(45) Bekanntmachung des Hinweises auf die Patenterteilung: **10.01.90 Patentblatt 90/2**

(84) Benannte Vertragsstaaten: **BE CH DE FR GB IT LI NL**

(56) Entgegenhaltungen:
**EP-A- 0 009 740**
**EP-A- 0 095 677**

(73) Patentinhaber: **BASF Aktiengesellschaft, Carl-Bosch-Strasse 38, D-6700 Ludwigshafen (DE)**

(72) Erfinder: **Rentzea, Costin, Dr., Richard-Kuhn-Strasse 1-3, D-6900 Heidelberg (DE)**
Erfinder: **Sauter, Hubert, Dr., Neckarpromenade, D-6800 Mannheim (DE)**
Erfinder: **Spiegler, Wolfgang, Dr., Westpreussenstrasse 5, D-6520 Worms 27 (DE)**
Erfinder: **Jung, Johann, Dr., Hardenburgstrasse 19, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 129 170 B1

**Beschreibung**

Die Erfindung betrifft neue Triazolyl-ketonoxime und -dioxime, Verfahren zu ihrer Herstellung, Mittel zur Regulierung des Pflanzenwachstums, die diese Verbindungen enthalten und Verfahren zur Regulierung des Pflanzenwachstums mit diesen Verbindungen.

Es ist bekannt, daß bestimmte 2-Halogenethyltrialkylammonium-halogenide pflanzenwachstumsregulierende Eigenschaften aufweisen (vgl. US-PS-3 156 554). So läßt sich mit Hilfe von (2-Chlorethyl)-trimethylammoniumchlorid das Pflanzenwachstum beeinflussen. Allerdings ist die Wirksamkeit dieses Stoffes, vor allem bei niedrigen Aufwandmengen nicht immer befriedigend.

Es ist ferner bekannt, 1-(4-Bromphenyl)-1-allyloxy-2-(1,2,4-triazolyl-(1)-ethan zur Regulierung des Pflanzenwachstums vor allem von Raps, Weizen, Hafer, Roggen und Gerste zu verwenden (DE-OS-2 650 831). Dessen Wirkung ist jedoch, vor allem bei niedrigen Aufwandmengen nicht immer befriedigend.

Es wurde nun gefunden, daß Verbindungen der Formel I

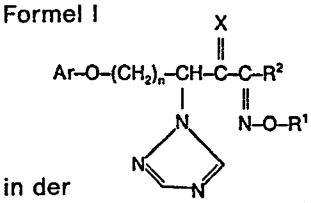

I,

in der

Ar einen Arylrest aus der Gruppe Biphenylyl, Naphthyl oder Phenyl, der durch Halogenatome, Nitro-, Cyan- oder Trifluormethylreste oder durch Alkyl-, Alkoxy- oder Alkenylreste mit jeweils bis zu 5 C-Atomen und ferner durch einen Phenoxyrest substituiert sein kann,

n ganze Zahlen von 2 bis 6,

X Sauerstoff oder einen $=N-OR^3$-Rest bedeutet, wobei $R^3$ ein Wasserstoffatom, einen gegebenenfalls durch Halogen oder Methoxy substituierten Alkyl-, Alkenyl- oder Alkinylrest mit jeweils bis zu 5 C-Atomen oder einen gegebenenfalls durch Halogenatome, Nitro-, Cyan-, Trifluormethyl, Alkyl- oder Alkoxyreste mit jeweils bis zu 4 C-Atomen substituierten Benzylrest oder einen $-CO-R^4$-Rest bedeutet, wobei $R^4$ einen gegebenenfalls durch Halogenatome oder Methoxy substituierten Alkylrest mit bis zu 5 C-Atomen, einen Phenylrest oder einen $-NH-R^5$-Rest bedeutet, wobei $R^5$ einen Alkylrest mit bis zu 4 C-Atomen oder einen gegebenenfalls chlorsubstituierten Phenylrest bedeutet,

$R^1$ einen gegebenenfalls durch einen Methoxyrest substituierten Alkylrest mit bis zu 8 C-Atomen oder einen gegebenenfalls durch Chlor oder Brom substituierten Benzyl- oder Phenylrest und

$R^2$ einen Alkylrest mit bis zu 8 C-Atomen bedeutet,

sowie deren für Pflanzen verträgliche Salze und Metallkomplexe hervorragend zur Beeinflussung des Pflanzenwachstums geeignet sind und eine sehr gute Pflanzenverträglichkeit besitzen.

Die neuen Verbindungen der Formel I können sterisch in der Z- oder in der E-Form vorliegen. In den meisten Fällen liegen Gemische vor. Die Z- und E-Isomeren lassen sich bei einigen der erfindungsgemäßen Verbindungen beispielsweise durch Löslichkeitsunterschiede oder durch Säulenchromatographie trennen und in reiner Form isolieren. Diese werden von der vorliegenden Erfindung ebenfalls umfaßt. Als Mittel zur Beeinflussung des Pflanzenwachstums kann man sowohl die einheitlichen geometrischen Isomeren wie auch deren Gemische verwenden. Aus wirtschaftlichen Gründen werden bevorzugt die letzteren verwendet, jedoch sind in manchen Fällen sterisch einheitliche Verbindungen mit einer günstigeren spezifischen Wirkung ausgestattet.

Ar bedeutet beispielsweise Biphenylyl-, 1- und 2-Naphthyl, bevorzugt aber Phenyl, 2- und 4-Fluorphenyl, 3- und 4-Chlorphenyl, 4-Bromphenyl, 4-Methoxyphenyl, 4-Ethoxyphenyl, 4-Propoxyphenyl, 4-Methylphenyl, 2,4-Dimethylphenyl, 4-Ethylphenyl, 4-Propylphenyl, 4-n-Butylphenyl, 4-Isobutylphenyl, 4-tert.-Butylphenyl, 4-Pentylphenyl, 4-Phenoxyphenyl, 4-Nitrophenyl, 3- und 4-Trifluormethylphenyl, 4-Cyanphenyl, 4-Allylphenyl, 3,4-Dichlorphenyl, 2,4-Dichlorphenyl und 2,6-Dichlorphenyl;

X bedeutet Sauerstoff oder einen Rest $=N-O-R^3$;

n bedeutet ganze Zahlen 2 bis 6, insbesondere 2 und 3;

$R^3$ bedeutet bevorzugt Wasserstoff, Methyl, Ethyl, n-Propyl, n-Butyl, Allyl, und ferner 2-Methoxyethyl, Isopropyl, Isobutyl, 2-Chlorallyl, 2-Chlorallyl, 2-Buten-1-yl, Pentenyl, Progargyl, Benzyl, 4-Fluorbenzyl, 4-Chlorbenzyl, 4-Trifluormethylbenzyl, 4-Nitrobenzyl, 4-Brombenzyl, 4-Methylbenzyl, 4-tert.-Butylbenzyl, 2,4-Dichlorbenzyl, 3,4-Dichlorbenzyl und 2,6-Dichlorbenzyl.

$R^4$ bedeutet bevorzugt Methyl, Ethyl, n-Propyl, i-Propyl, ferner beispielsweise Chlormethyl, Brommethyl, Methoxymethyl, 2-Chlorethyl und Phenyl;

$R^5$ bedeutet beispielsweise Methyl, Ethyl, Propyl, n-Butyl, Phenyl oder 4-Chlorphenyl;

$R^1$ bedeutet bevorzugt Methyl, Ethyl und ferner beispielsweise 2-Methoxyethyl, n-Propyl, Isopropyl, n-Butyl, Isobutyl, n-Pentyl, Isopentyl, n-Hexyl, n-Octyl, Benzyl, 4-Chlorbenzyl, 2,4-Dichlorbenzyl, 4-Brombenzyl und 2-Phenylethyl;

$R^2$ bedeutet bevorzugt Methyl, Ethyl, n-Propyl, und ferner beispielsweise Isopropyl, n-Butyl, Isobutyl, tert.-Butyl, n-Pentyl und n-Heptyl.

Triazolyl-ketonoxime und -dioxime der Formel I erhält man, wenn man ein Ketonoxim der Formel

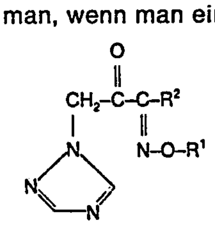

II,

in der $R^1$ und $R^2$ die oben genannten Bedeutungen haben oder dessen Alkalienolat mit einem Aryloxyalkylhalogenid der Formel

$$Ar-O-(CH_2)_n-Y \qquad III$$

in dem Ar und n die oben angegebenen Bedeutungen haben und Y Chlor, Brom oder Iod bedeutet, umsetzt und die so erhaltene Verbindung der Formel I, in der X zunächst ein Sauerstoffatom bedeutet, mit Hydroxylamin oder mit dessen Salzen zu einem Oxim der Formel I, in der X eine =N–OH-Gruppe bedeutet, umsetzt und das so erhaltene Dioxim mit einem Alkylierungsmittel der Formel

$$R^3–Y, \qquad IV$$

einem Säurechlorid der Formel $R^4$–COCl, einem Säureanhydrid der Formel $(R^4–CO)_2O$ oder ferner mit einem Isocyanat der Formel $R^5$–N=C=O oder mit einem Carbamoylchlorid der Formel $R^5$–NH–COCl, in denen jeweils $R^3$, $R^4$ und $R^5$ die oben angegebenen Bedeutungen haben und Y Chlor, Brom oder Iod bedeutet, gegebenenfalls in Gegenwart eines Verdünnungsmittels und/oder einer anorganischen oder organischen Base und/oder eines Reaktionsbeschleunigers und/oder eines Phasentransferkatalysators bei einer ausreichenden Temperatur von bis zu 100 °C umsetzt und die so erhaltene Verbindung – falls gewünscht – mit Säure in ihre Säureadditionssalze oder mit Metallsalzen in ihre Metallkomplexe überführt.

Zur Herstellung von Triazolyl-dioximen der Formel I gemäß Anspruch 1, in der X einen Rest =N–O–$R^3$ und wobei $R^3$ verschieden von H ist bedeutet, können auch Triazolyl-ketonoxime der Formel I gemäß Anspruch 1, in der X Sauerstoff bedeutet, mit Hydroxylamin oder mit einem Hydroxylamin-Derivat der Formel $H_2N–O–R^3$, in der $R^3$ die im Anspruch 1 angegebene Bedeutung hat, gegebenenfalls in Gegenwart eines basischen oder sauren Katalysators und gegebenenfalls in Gegenwart eines Verdünnungsmittels umgesetzt werden.

Die Umsetzung des Ketonoxims (II) mit dem Aryloxyalkylhalogenid (III) nimmt man vorteilhaft in einem Lösungs- oder Verdünnungsmittel und in Gegenwart einer Base, vorzugsweise nach Zusatz einer starken anorganischen Base bei einer ausreichenden Temperatur i. a. unter 120 °C, bevorzugt zwischen 0 und 100 °C vor. Bevorzugt wird ein aprotisch-polares Lösungsmittel wie Dimethylformamid, Diethylformamid, Dimethylacetamid, Diethylacetamid, N-Methylpyrrolidon, Hexamethyl-phosphorsäuretriamid, Dimethylsulfoxid und Tetrahydrothiophen-1,1-dioxid (Sulfolan).

Geeignete Basen bzw. säurebindende Mittel sind beispielsweise Alkalihydride, wie Lithium-, Natrium- und Kaliumhydrid, Alkaliamide wie Natrium- und Kaliumamid oder Natrium- bzw. Kaliumdiisopropylamid, Alkalialkoholate wie Natrium- oder Kaliummethoxid, -ethoxid oder tert.-butoxid, Lithium-, Natrium- oder Kalium-Triphenylmethyl und Naphthalin-Lithium, -Natrium oder Kalium.

Ketonoxime (II) sind ebenfalls neue Stoffe und besitzen, neben ihrer Eigenschaft als wertvolle Zwischenprodukte, wachstumsregulierende Wirkung. Sie können, wie sich für den Fachmann bei Kenntnis ihrer Strukturformel ergibt, durch Umsetzung von bekannten 1-Halogen-3-oximino-3-alkyl-propan-2-onen (V)

$$Y–CH_3–CO–\overset{\overset{\displaystyle N–OR^1}{\|}}{C}–R_2 \qquad (V)$$

mit 1,2,4-Triazol erhalten werden. Eine geeignete Arbeitsvorschrift findet sich bei den Herstellungsbeispielen.

Die Umsetzung der Verbindungen der Formel I, in denen X Sauerstoff bedeutet, mit Hydroxylamin bzw. Hydroxylamin-Derivaten bzw. deren Salzen wird zweckmäßig in Wasser oder in einem mit Wasser mischbaren Lösungsmittel durchgeführt. Es eignen sich hierfür beispielsweise Alkohole, wie Methanol, Ethanol, Propanol, Ethylenglykol, 2-Methoxyethanol; Ether wie Tetrahydrofuran und Dioxan, Säureamide, wie Dimethylformamid, Diethylformamid, Dimethylacetamid, ferner N-Methyl-pyrrolidon und Hexamethyl-phosphorsäuretriamid oder Carbonsäuren wie Ameisensäure, Essigsäure und Propionsäure. Wenn ein neutrales Lösungsmittel verwendet wird, kann es zweckmäßig sein, eine Base wie beispielsweise Natriumhydroxid, Kaliumhydroxid, Bariumhydroxid, Natriumacetat, ein Amin wie Triethylamin, Piperidin und N-Methylpiperidin, eine Mineralsäure wie Salzsäure, Schwefelsäure, Phosphorsäure oder eine Carbonsäure wie Ameisensäure oder Essigsäure zuzusetzen.

Die Umsetzung verläuft im allgemeinen unterhalb von 80 °C, meist zwischen 0 und 40 °C.

Die Umsetzung des Triazolyl-dioxims der Formel I (X = N–OH) mit den Alkylierungsmitteln, Säurechlorid bzw. -anhydrid, Isocyanat oder Carbamoylchlorid kann ohne Verdünnungsmittel oder in einem geeigneten Lösungsmittel stattfinden. Ein geeignetes Lösungsmittel ist beispielsweise Acetonitril; ein Ether wie Diethyl-, Dipropyl-, Dibutyl- oder Methyl-tert.-butylether, Tetrahydrofuran, Dioxan und Dimethoxyethan; ein Ester wie Essigsäureethylester; ein Keton wie Aceton und Methylethylketon; ein chlorierter Kohlenwasserstoff wie Methylenchlorid, Chloroform, Tetrachlorkohlenstoff, Dichlorethan, 1,1,1-Trichlorethan, Tetrachlorethan, Chlorbenzol; ein Kohlenwasserstoff wie Benzol, Toluol und Xylol. Dabei ist es zweckmäßig, eine Base, beispielsweise ein Alkali- oder Erdalkalihydroxid wie Natriumhydroxid, Kaliumhydroxid, Calciumhydroxid, Bariumhydroxid, ein Alkali- oder Erdalkalicarbonat wie Natriumcarbonat, Kaliumcarbonat, Natriumhydrogencarbonat, Kaliumhydrogencarbonat, Calciumcarbonat, Bariumcarbonat, ein Alkali- oder ein Erdalkalialkoholat wie Natriummethylat, Natriumethylat, Magnesiummethylat, Natriumisopropylat oder Kalium-tert.-butylat, ein Amin wie Triethylamin, N,N-Dimethylcyclohexylamin, Piperidin, N-Methylpiperidin oder Pyridin zuzusetzen.

Geeignet als Reaktionsbeschleuniger sind beispielsweise Metallhalogenide wie Natriumbromid, Natriumiodid, Kaliumbromid, Kaliumiodid, tertiäre Amine wie 4-Dimethylaminopyridin oder 4-Pyrrolidinopyridin, Kronenether wie 12-Krone-4, 14-Krone-5, 18-Krone-6, Dibenzo-18-Krone-6 oder Dicyclohexano-18-Krone-6 oder Azole wie Imidazol und 1,2,4-Triazol.

Als Phasentransferkatalysatoren können quaternäre Ammoniumsalze wie Tetrabutylammoniumbromid, -bromid, -iodid oder -hydrogensulfat, Benzyltriethylammoniumchlorid, Methyl-trioctylammoniumchlorid und -bromid und Phosphoniumsalze wie Tetrabutylphosphoniumbromid und -iodid und Tetra-n-pentyl-phosphoniumbromid und -iodid dienen.

Die Verbindungen der Formel I werden mit üblichen Methoden isoliert, gegebenenfalls gereinigt und gegebenenfalls mit Säuren oder Metallsalzen zu Salzen bzw. zu Metall-Komplexen umgesetzt.

Beispiel 1

a) Herstellung eines Vorproduktes:

Zu einer unter reinem Stickstoff gerührten Suspension von 68,2 g (0,75 Mol) Natrium-1,2,4-triazolid in 250 ml trockenem Tetrahydrofuran wird die Lösung von 144 g (0,74 Mol) 1-Brom-3-methoxyimino-butan-2-on (T. Wieland u. J. Stärke, Chem. Ber., 96, 2410 (1963)) in 200 ml Tetrahydrofuran bei 25 °C in 2 Stunden zugetropft. Das Gemisch wird 2 Tage bei 25 °C und dann 3 Stunden bei 60 °C nachgerührt. Der entstandene Niederschlag wird abfiltriert und das Filtrat unter vermindertem Druck eingeengt. Man löst in 300 ml Methylenchlorid, wäscht dreimal mit je 50 ml Wasser, trocknet über Natriumsulfat und engt ein. Der Rückstand wird mit 80 ml Ether verrührt und über Nacht bei +3 °C aufbewahrt, der Niederschlag abgesaugt, mit 30 ml kaltem (+5 °C) Ether, dann mit 100 ml n-Pentan gewaschen und anschließend getrocknet.

Es wurden 56 g (41% d. Th.) 1-(1,2,4-Triazolyl-(1))-3-methoxyimino-butan-2-on als blaßgelbe Kristalle vom Schmp. 87 bis 89 °C erhalten.

b) Herstellung des Endproduktes

Zu einer unter reinem Stickstoff gerührten Suspension von 5 g (0,21 Mol) Natriumhydrid in 100 ml trockenem Dimethylformamid wird eine Lösung von 36,4 g (0,2 Mol) des nach der vorstehenden Vorschrift erhaltenen 1-(1,2,4-Triazolyl-(1))-3-methoxyimino-butan-2-ons in 50 ml Dimethylformamid bei 20 bis 25 °C zugetropft.

Nach dreistündigem Nachrühren wird bei 25 °C die Lösung von 40,2 g (0,2 Mol) 2-Phenoxyethylbromid in 35 ml Dimethylformamid zugetropft und weitere 15 Stunden nachgerührt. 40 ml Eiswasser werden vorsichtig zugetropft und das Gemisch i. V. eingeengt. Der Rückstand wird zwischen 250 ml Methylenchlorid und 80 ml Wasser verteilt, die organische Phase dreimal mit je 100 ml Wasser gewaschen, über $Na_2SO_4$ getrocknet und i. V. – schließlich bis 70 °C und 20 Pa (0,2 mbar) eingeengt. Es wurden 45,9 g (76, % d. Th.)

1-Phenoxy-3-(1,2,4-triazolyl-(1))-5-methoxyimino-hexan-4-on

als hellbraunes Harz erhalten.

IR (Film: 1706, 1600, 1588, 1500, 1277, 1244, 1175, 1140, 1048, 888, 756, 692 und 678 $cm^{-1}$

Beispiel 2

Zu einer Lösung von 100 g (1,44 Mol) Hydroxylaminhydrochlorid in 1200 ml Ethanol werden 244 g (1,45 Mol) einer 30%igen Natriummethylatlösung in Methanol bei 20 °C zugetropft. Anschließend wird die Lösung von 241 g (0,8 Mol) des gemäß Beispiel 1 gewonnenen 1-Phenoxy-3-(1,2,4-triazolyl-(1))-5-methoxyimino-hexan-4-on in 300 ml Ethanol zugegeben, das Gemisch 3 Tage bei 70 °C gerührt, dann auf 20 °C abgekühlt. Der organische Niederschlag wird abgesaugt und das Filtrat i. V. eingeengt. Der Rückstand wird zwischen 1000 ml Essigester und 300 ml einer 5%igen Natriumcarbonatlösung verteilt, die organische Phase zweimal mit je 100 ml Wasser gewaschen, über $Na_2SO_4$ getrocknet und eingeengt. Den Rückstand läßt man über Nacht mit 100 ml Ether bei +3 °C stehen, saugt ab, wäscht mit je 50 ml eiskaltem Ether und n-Pentan und trocknet.

Es wurden 106,5 g (42% d. Th.) 1-Phenoxy-3-(1,2,4-triazolyl-(1))-4-hydroxyimino-5-methoxyimino-hexan als farblose Kristalle vom Schmp. 133 bis 137 °C erhalten.

Beispiel 3

Zu einer Lösung von 15,2 g (0,048 Mol)

1-Phenoxy-3-(1,2,4-triazolyl(1))-4-hydroxyimino-5-methoxyimino-hexan,

15 ml Pyridin und 0,5 g 4-Dimethylaminopyridin in 50 ml Methylenchlorid wird die Lösung von 7,7 g (0,055 Mol) Benzoylchlorid bei 10 bis 20 °C zugetropft. Das Gemisch wird über Nacht bei Raumtemperatur nachgerührt und anschließend mit 150 ml Methylenchlorid und 100 ml Wasser versetzt. Die organische Phase wird dreimal mit je 50 ml Wasser gewaschen, über $Na_2SO_4$ getrocknet und eingeengt.

Es wurden 16,5 g (81,7% d. Th.)

1-Phenoxy-3-(1,2,4-triazolyl-(1))-4-benzoyloxyimino-5-methoxyimino-hexan

als hellbraunes Harz erhalten.

IR (Film): 1760, 1601, 1500, 1451, 1274, 1241, 1046, 1020, 707 und 678 $cm^{-1}$

Beispiel 4

Zu einer Lösung von 15,2 g (0,048 Mol)

1-Phenoxy-3-(1,2,4-triazolyl-(1))-4-hydroxyimino-5-methoxyimino-hexan

in 80 ml Methanol werden 9,4 g (0,052 Mol) einer 30%igen Natriummethylatlösung in Methanol bei 20 °C zugetropft. Anschließend wird die Lösung von 9,6 g (0,052 Mol) Benzylbromid in 15 ml Tetrahydrofuran zugegeben, das Gemisch 15 Stunden bei 20 °C gerührt und eingeengt. Der Rückstand wird zwischen 250 ml Ether und 70 ml Wasser verteilt, die organische Phase dreimal mit je 50 ml Wasser gewaschen, über $Na_2SO_4$ getrocknet und schließlich bei 70 °C und 0,2 mbar eingeengt.

Es wurden 16,8 g (86% d. Th.)

1-Phenoxy-3-(1,2,4-triazolyl-(1))-4-benzyloxyimino-5-methoxyimino-hexan

als gelbliches Harz erhalten.

IR (Film): 1599, 1587, 1498, 1471, 1364, 1274, 1244, 1138, 1048, 1027, 1016, 892, 755 und 678 $cm^{-1}$

Durch entsprechende Abwandlung der vorstehenden Herstellbeispiele wurden die in der folgenden Tabelle aufgeführten Verbindungen erhalten.

Tabelle

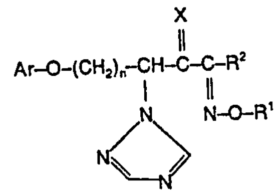

I,

| Nr. | Ar | X | $R^1$ | $R^2$ | n | Phys. Konstante oder IR-Spektr. [$cm^{-1}$] (Film) |
|---|---|---|---|---|---|---|
| 5 | $C_6H_5-$ | $C_2H_5CO-O-N=$ | $CH_3$ | $CH_3$ | 2 | 1781, 1600, 1590, 1500, 1245, 1142, 1116, 1049, 1007, 896, 756, 695. |
| 7 | " | 0 | $C_2H_5$ | $CH_3$ | 2 | 1706, 1600, 1588, 1499, 1277, 1244, 1140, 1044, 1008, 756, 694, 677. |
| 8 | " | $=N-OH$ | $C_2H_5$ | $CH_3$ | 2 | Schmp. 115–118°C |
| 9 | " | $C_2H_5CO-O-N=$ | $C_2H_5$ | $CH_3$ | 2 | 1781, 1600, 1500, 1245, 1174, 1140, 1118, 1046, 1007, 888, 756, 692, 678. |
| 10 | " | 0 | $CH_3$ | $CH_3$ | 3 | 1705, 1601, 1586, 1499, 1276, 1245, 1050, 756, 692, 678. |
| 11 | $C_6H_5-$ | $=N-OH$ | $CH_3$ | $CH_3$ | 3 | Schmp. 128–130°C |
| 12 | " | $C_2H_5CO-O-N=$ | $CH_3$ | $CH_3$ | 3 | 1780, 1600, 1588, 1500, 1246, 1173, 1141, 1117, 1048, 1007, 883, 756. |
| 13 | " | $4-CH_3-C_6H_4-CH_2-O-N=$ | $CH_3$ | $CH_3$ | 3 | 1599, 1587, 1498, 1275, 1245, 1049, 1013, 895, 755, 692. |
| 14 | " | 0 | $CH_3$ | $CH_3$ | 4 | 1705, 1600, 1589, 1499, 1478, 1277, 1246, 1051, 756, 693, 578. |
| 15 | " | $=N-OH$ | $CH_3$ | $CH_3$ | 4 | Schmp. 130–132°C |
| 16 | " | $C_2H_5CO-O-N=$ | $CH_3$ | $CH_3$ | 4 | 1781, 1600, 1587, 1500, 1276, 1246, 1173, 1142, 1117, 1049, 1009, 893, 757, 692. |
| 17 | " | $4-Cl-C_6H_4-CH_2-O-N=$ | $CH_3$ | $CH_3$ | 4 | 1586, 1497, 1472, 1276, 1245, 1140, 1089, 1049, 1012, 888, 802, 755, 692, 678. |
| 18 | $4-F-C_6H_4-$ | 0 | $CH_3$ | $CH_2$ | 2 | 1706, 1507, 1277, 1248, 1209, 1139, 1098, 1048, 829, 678. |
| 19 | $4-F-C_6H_4-$ | $=N-OH$ | $CH_3$ | $CH_3$ | 2 | Schmp. 156–159°C |
| 20 | " | $C_2H_5CO-O-N=$ | $CH_3$ | $CH_3$ | 2 | 1781, 1507, 1247, 1208, 1139, 1049, 1005, 973, 894, 876, 829. |
| 21 | " | $C_6H_5-CH_2-O-N=$ | $CH_3$ | $CH_3$ | 2 | 1507, 1247, 1208, 1050, 1017, 908, 887, 828, 756, 742, 700. |
| 22 | " | 0 | $C_2H_5$ | $CH_3$ | 2 | 1706, 1507, 1277, 1248, 1209, 1139, 1044, 1008, 829, 748, 678. |
| 23 | " | $=N-OH$ | $C_2H_5$ | $CH_3$ | 2 | Schmp. 139°C |
| 24 | " | $C_2H_5CO-O-N=$ | $C_2H_5$ | $CH_3$ | 2 | 1781, 1507, 1275, 1248, 1208, 1139, 1097, 1052, 830, 678. |
| 26 | " | $=N-OH$ | $CH_3$ | $CH_3$ | 3 | Schmp. 113–114°C |
| 27 | " | $C_2H_5CO-O-N=$ | $CH_3$ | $CH_3$ | 3 | 1780, 1507, 1275, 1249, 1207, 1140, 1118, 1048, 1008, 880, 830, 680. |
| 28 | $4-F-C_6H_4-$ | $C_2H_5-O-N=$ | $CH_3$ | $CH_3$ | 3 | 1507, 1473, 1276, 1247, 1208, 1139, 1043, 985, 956, 829, 678. |
| 29 | " | $n-C_3H_7-O-N=$ | $CH_3$ | $CH_3$ | 3 | 1507, 1474, 1277, 1208, 1098, 1049, 1024, 955, 937, 829, 678. |
| 30 | " | 0 | $C_2H_5$ | $CH_3$ | 3 | 1705, 1507, 1277, 1248, 1209, 1097, 1044, 1009, 829, 678. |
| 31 | " | $=N-OH$ | $C_2H_5$ | $CH_3$ | 3 | Schmp. 124–127°C |
| 32 | " | $C_2H_5CO-O-N=$ | $C_2H_5$ | $CH_3$ | 3 | 1781, 1507, 1276, 1248, 1207, 1141, 1119, 1046, 1008, 885, 830. |
| 33 | " | $4\ CF_3-C_6H_4-CH_2-O-N=$ | $C_2H_5$ | $CH_3$ | 3 | 1507, 1325, 1277, 1248, 1208, 1165, 1126, 1066, 1047, 1015, 956, 828, 678. |
| 34 | " | 0 | $CH_3$ | $CH_3$ | 4 | 1705, 1507, 1277, 1248, 1209, 1140, 1050, 1016, 829, 678. |
| 35 | " | $=N-OH$ | $CH_3$ | $CH_3$ | 4 | Schmp. 114–116°C |
| 36 | " | $4-(tert.-C_4H_9)-C_6H_4-CH_2-O-N=$ | $CH_3$ | $CH_3$ | 4 | Harz |
| 37 | $4-F-C_6H_4-$ | $C_2H_5CO-O-N=$ | $CH_3$ | $CH_3$ | 4 | 1782, 1507, 1464, 1248, 1207, 1142, 1117, 1049, 1009, 894, 831, 680. |
| 38 | $2-C_{10}H_7$ | 0 | $CH_3$ | $CH_3$ | 2 | Harz |
| 39 | " | $=N-OH$ | $CH_3$ | $CH_3$ | 2 | Schmp. 155–158°C |

Tabelle (Fortsetzung)

| Nr. | Ar | X | $R^1$ | $R^2$ | n | Phys. Konstante oder IR-Spektr. [$cm^{-1}$] (Film) |
|---|---|---|---|---|---|---|
| 40 | " | $C_2H_5CO$–O–N= | $CH_3$ | $CH_3$ | 2 | 1780, 1630, 1505, 1273, 1258, 1217, 1182, 1138, 1121, 1051, 1006, 890, 840, 750, 680. |
| 41 | 4 Cl–$C_6H_4$– | 0 | $CH_3$ | $CH_3$ | 2 | Harz |
| 42 | " | =N–OH | $CH_3$ | $CH_3$ | 2 | Schmp. 161–163°C |
| 43 | 3 Cl–$C_6H_4$– | 0 | $CH_3$ | $CH_3$ | 2 | 1707, 1595, 1581, 1503, 1481, 1283, 1248, 1231, 1140, 1052, 940, 860, 775, 679. |
| 44 | " | =N–OH | $CH_3$ | $CH_3$ | 2 | Schmp. 130–135°C |
| 45 | 4 Br–$C_6H_4$– | 0 | $CH_3$ | $CH_3$ | 2 | 1706, 1592, 1502, 1489, 1279, 1243, 1174, 1049, 1004, 824, 678, 644. |
| 46 | " | =N–OH | $CH_3$ | $CH_3$ | 2 | Schmp. 134–137°C |
| 47 | 4 Br–$C_6H_4$– | $C_2H_5CO$–O–N= | $CH_3$ | $CH_3$ | 2 | 1779, 1489, 1277, 1243, 1173, 1140, 1116, 1048, 1003, 824, 680. |
| 48 | 4 $CH_3$–$C_6H_4$– | 0 | $CH_3$ | $CH_3$ | 2 | Harz |
| 49 | " | =N–OH | $CH_3$ | $CH_3$ | 2 | Schmp. 137–139°C |
| 50 | " | $C_2H_5CO$–O–N= | $CH_3$ | $CH_3$ | 2 | 1782, 1512, 1277, 1242, 1140, 1120, 1051, 1013, 894, 678. |
| 51 | 3 $CH_3$–$C_6H_4$– | 0 | $CH_3$ | $CH_3$ | 2 | 1707, 1603, 1585, 1502, 1491, 1276, 1263, 1159, 1051, 778, 690, 678. |
| 52 | " | =N–OH | $CH_3$ | $CH_3$ | 2 | Schmp. 116–119°C |
| 53 | 3 $F_3C$–$C_6H_4$– | 0 | $CH_3$ | $CH_3$ | 2 | 1706, 1502, 1450, 1330, 1278, 1239, 1169, 1126, 1066, 1050, 795. |
| 54 | " | =N–OH | $CH_3$ | $CH_3$ | 2 | Schmp. 146–148°C |
| 55 | " | $C_2H_5$–CO–O–N= | $CH_3$ | $CH_3$ | 2 | 1782, 1504, 1456, 1330, 1239, 1169, 1126, 1067, 1050, 1007, 895, 795, 700. |
| 56 | 4 $CH_3O$–$C_6H_4$– | 0 | $CH_3$ | $CH_3$ | 2 | 1705, 1509, 1277, 1232, 1140, 1046, 1008, 827, 678. |
| 57 | 4 $CH_3O$–$C_6H_4$– | =N–OH | $CH_3$ | $CH_3$ | 2 | Schmp. 104–106°C |
| 58 | " | 4 Br–$C_6H_4$–$CH_2$–O–N= | $CH_3$ | $CH_3$ | 2 | 1593, 1508, 1488, 1466, 1232, 1070, 1047, 1009, 887, 824, 797, 678. |
| 59 | 3 $No_2$–$C_6H_4$– | 0 | $CH_3$ | $CH_3$ | 2 | Schmp. 142–144°C |
| 60 | " | =N–OH | $CH_3$ | $CH_3$ | 2 | Schmp. 175–177°C |
| 61 | 3,4 $Cl_2$–$C_6H_3$– | 0 | $CH_3$ | $CH_3$ | 2 | 1707, 1594, 1503, 1478, 1468, 1365, 1295, 1278, 1231, 1136, 1124, 1050, 1024, 1007, 676. |
| 62 | " | =N–OH | $CH_3$ | $CH_3$ | 2 | Schmp. 119–121°C |
| 63 | " | $C_2H_5CO$–O–N= | $CH_3$ | $CH_3$ | 2 | 1780, 1594, 1503, 1468, 1278, 1136, 1124, 1051, 1006, 973, 893, 678. |
| 64 | $C_6H_5$ | 0 | $CH_3$ | n–$C_3H_7$ | 2 | 1710, 1600, 1499, 1275, 1244, 1047, 1020, 756, 678. |
| 65 | " | =N–OH | $CH_3$ | n–$C_3H_7$ | 2 | |
| 66 | " | 0 | $CH_3$ | iso–$C_4H_9$ | 2 | 1709, 1600, 1588, 1499, 1276, 1245, 1338, 1048, 756, 692. |
| 67 | "(Isomer 1) | =N–OH | $CH_3$ | iso-$C_4H_9$ | 2 | Schmp. 148–150°C |
| 68 | $C_6H_5$(Isomer 2) | =N–OH | $CH_3$ | iso-$C_4H_9$ | 2 | 1600, 1499, 1465, 1278, 1244, 1338, 1047, 1018, 908, 755. |
| 69 | " | 0 | $CH_3$ | iso–$C_4H_9$ | 4 | 1707, 1600, 1499, 1470, 1276, 1245, 1138, 1047, 755. |
| 71 | " | 0 | $CH_3$ | n–$C_8H_{17}$ | 4 | Harz |
| 72 | " | =N–OH | $CH_3$ | n–$C_8H_{17}$ | 4 | Schmp. 136–140°C |
| 73 | 4–$FC_6H_4$– | 0 | $CH_3$ | iso-$C_4H_9$ | 2 | 1709, 1507, 1275, 1248, 1210, 1047, 1020, 829, 678. |
| 74 | "(Isomer 1) | =N–OH | $CH_3$ | iso–$C_4H_9$ | 2 | Schmp. 78–81°C |
| 75 | "(Isomer 2) | =N–OH | $CH_3$ | iso–$C_4H_9$ | 2 | 1509, 1282, 1212, 1139, 1050, 1026, 1004, 907, 830. |

Die neuen Verbindungen können praktisch alle Entwicklungsstadien einer Pflanze verschiedenartig beeinflussen und werden deshalb als Wachstumsregulatoren eingesetzt. So wird man ein oder mehrere Verbindungen gemäß den Ansprüchen 1, 2 oder 3 auf Pflanzen oder deren Lebensraum einwirken lassen, um das Pflanzenwachstum zu regulieren.

Durch Mischen der erfindungsgemäßen Verbindungen gemäß den Ansprüchen 1, 2 oder 3 mit üblichen Zusatzstoffen wie festen oder flüssigen Trägerstoffen sowie gegebenenfalls einem oder mehreren oberflächenaktiven Stoffen werden das Pflanzenwachstum regulierende Mittel hergestellt.

Solche Mittel, enthaltend ein oder mehrere Verbindungen gemäß den Ansprüchen 1, 2 oder 3, sind somit ebenfalls Gegenstand der Erfindung.

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Prüfsubstanzen (Wirkstoffe) in wäßriger Aufbereitung am Tage der Einsaat auf das Saatbett gegossen.

Im Nachauflaufverfahren wurden die zu prüfenden Substanzen in wäßriger Zubereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleich diente Chlorcholinchlorid (CCC).

Mit der Reduzierung des Längenwachstums nahm die Farbintensität der Blätter zu, was auf erhöhten Chlorophyllgehalt schließen läßt und letztlich über eine erhöhte Photosyntheserate eine erhöhte Ertragsbildung erwarten läßt.

Tabelle 1
Sommergerste «Union», Vorlauflaufverfahren

| Beispiel | Konzentration mg Wirkstoff/Gefäß | relative Wuchshöhe |
|---|---|---|
| unbehandelt | – | 100 |
| CCC | 3 | 90,2 |
| | 12 | 76,5 |
| 2 | 3 | 56,6 |
| | 12 | 43,4 |
| 5 | 3 | 94,1 |
| | 12 | 58,8 |
| 20 | 3 | 83,6 |
| | 12 | 53,5 |
| 37 | 3 | 87,0 |
| | 12 | 80,3 |
| 30 | 3 | 90,3 |
| | 12 | 83,6 |
| 7 | 3 | 93,6 |
| | 12 | 76,9 |
| 22 | 3 | 93,6 |
| | 12 | 60,2 |
| 27 | 3 | 83,6 |

Tabelle 1 (Fortsetzung)
Sommergerste «Union», Vorlauflaufverfahren

| Beispiel | Konzentration mg Wirkstoff/Gefäß | relative Wuchshöhe |
|---|---|---|
| | 12 | 33,4 |
| 12 | 3 | 87,0 |
| | 12 | 33,4 |
| 19 | 3 | 85,3 |
| | 12 | 50,2 |
| 26 | 3 | 87,0 |
| | 12 | 53,5 |
| 11 | 3 | 76,9 |
| | 12 | 43,5 |
| 32 | 3 | 90,2 |
| | 12 | 64,7 |

Tabelle 2
Sonnenblumen «Sobrid», Nachauflaufverfahren

| Beispiel | Konzentration mg Wirkstoff/Gefäß | relative Wuchshöhe |
|---|---|---|
| unbehandelt | – | 100 |
| CCC | 1,5 | 90,2 |
| | 6 | 92,0 |
| 2 | 1,5 | 82,7 |
| | 6 | 71,2 |
| 52 | 1,5 | 85,3 |
| | 6 | 82,0 |
| 44 | 1,5 | 85,3 |
| | 6 | 83,7 |
| 50 | 1,5 | 80,0 |
| | 6 | 76,9 |
| 27 | 1,5 | 89,0 |
| | 6 | 78,3 |
| 12 | 1,5 | 81,9 |
| | 6 | 74,7 |
| 26 | 1,5 | 78,3 |
| | 6 | 74,7 |
| 11 | 1,5 | 85,4 |
| | 6 | 74,7 |

Tabelle 3
Sommerraps, «Petranova», Vorauflaufverfahren

| Beispiel | Konzentration mg Wirksoff/Gefäß | relative Wuchshöhe |
|---|---|---|
| unbehandelt | – | 100 |
| CCC | 3 | 100 |
| | 12 | 93,8 |
| 49 | 3 | 76,3 |
| | 12 | 67,8 |
| 2 | 3 | 61,4 |
| | 12 | 22,7 |
| 46 | 3 | 80,0 |
| | 12 | 75,0 |

Tabelle 3 (Fortsetzung)
Sommerraps, «Petranova», Vorauflaufverfahren

| Beispiel | Konzentration mg Wirksoff/Gefäß | relative Wuchshöhe |
|---|---|---|
| 5 | 3 | 59,3 |
| | 12 | 35,6 |
| 50 | 3 | 83,0 |
| | 12 | 75,1 |
| 20 | 3 | 31,3 |
| | 12 | 27,8 |
| 30 | 3 | 72,9 |
| | 12 | 66,0 |
| 7 | 3 | 79,9 |
| | 12 | 79,9 |
| 22 | 3 | 72,9 |
| | 12 | 17,4 |
| 12 | 3 | 48,6 |
| | 12 | 20,8 |
| 19 | 3 | 38,2 |
| | 12 | 38,2 |
| 26 | 3 | 76,4 |
| | 12 | 41,7 |
| 11 | 3 | 69,4 |
| | 12 | 52,1 |

Tabelle 4
Sommerraps «Petranova», Nachauflaufverfahren

| Beispiel | Konzentration mg Wirkstoff/Gefäß | relative Wuchshöhe |
|---|---|---|
| unbehandelt | – | 100 |
| CCC | 1,5 | 88,4 |
| | 6 | 86,3 |
| 49 | 1,5 | 90,1 |
| | 6 | 73,0 |
| 42 | 1,5 | 90,1 |
| | 6 | 73,0 |
| 59 | 1,5 | 90,1 |
| | 6 | 68,7 |
| 2 | 1,5 | 78,4 |
| | 6 | 58,8 |
| 46 | 1,5 | 84,5 |
| | 6 | 70,0 |
| 52 | 1,5 | 76,5 |
| | 6 | 58,8 |
| 44 | 1,5 | 82,4 |
| | 6 | 54,9 |
| 60 | 1,5 | 82,4 |
| | 6 | 78,4 |
| 5 | 1,5 | 57,4 |
| | 6 | 55,6 |
| 47 | 1,5 | 68,5 |
| | 6 | 48,1 |
| 50 | 1,5 | 59,3 |
| | 6 | 48,1 |
| 20 | 1,5 | 72,8 |
| | 6 | 63,2 |
| 27 | 1,5 | 72,8 |
| | 6 | 67,0 |

Tabelle 4 (Fortsetzung)
Sommerraps «Petranova», Nachauflaufverfahren

| Beispiel | Konzentration mg Wirkstoff/Gefäß | relative Wuchshöhe |
|---|---|---|
| 63 | 1,5 | 69,0 |
| | 6 | 55,6 |
| 12 | 1,5 | 61,3 |
| | 6 | 57,5 |
| 55 | 1,5 | 76,6 |
| | 6 | 57,7 |
| 11 | 1,5 | 70,9 |
| | 6 | 59,4 |
| 62 | 1,5 | 74,7 |
| | 6 | 65,1 |
| 24 | 1,5 | 74,3 |
| | 6 | 54,3 |
| 32 | 1,5 | 70,3 |
| | 6 | 62,2 |
| 31 | 1,5 | 86,3 |
| | 6 | 66,3 |
| 23 | 1,5 | 88,4 |
| | 6 | 60,2 |
| 21 | 1,5 | 54,1 |
| | 6 | 47,6 |
| 13 | 1,5 | 80,1 |
| | 6 | 60,6 |
| 66 | 1,5 | 76,7 |
| | 6 | 69,8 |
| 69 | 1,5 | 76,7 |
| | 6 | 74,4 |
| 73 | 1,5 | 74,4 |
| | 6 | 67,4 |
| 75 | 1,5 | 78,2 |
| | 6 | 66,3 |

**Patentansprüche**

1. Triazolyl-ketonoxim und -dioxim der allgemeinen Formel I

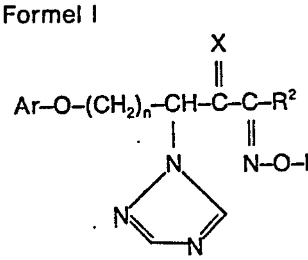

I,

in der

Ar einen Arylrest aus der Gruppe Biphenyl, Naphthyl oder Phenyl, der durch Halogenatome, Nitro- Cyan oder Trifluormethylreste oder durch Alkyl-, Alkoxy- oder Alkenylreste mit jeweils bis zu 5 C-Atomen und ferner durch einen Phenoxyrest substituiert sein kann,

n ganze Zahlen von 2 bis 6,

X Sauerstoff oder einen $=N-OR^3$-Rest bedeutet, wobei $R^3$ ein Wasserstoffatom, einen gegebenenfalls durch Halogen substituierten Alkyl-, Alkenyl- oder Alkinylrest mit jeweils bis zu 5 C-Atomen oder einen gegebenenfalls durch Halogenatome,

Nitro-, Cyan-, Trifluormethyl, Alkyl- oder Alkoxyreste mit jeweils bis zu 4 C-tomen substituierten Benzylrest oder einen $-CO-R^4$-Rest bedeutet, wobei $R^4$ einen gegebenenfalls durch Halogenatome substituierten Alkylrest mit bis zu 5 C-Atomen, einen Phenylrest oder einen $-NH-R^5$-Rest bedeutet, wobei $R^5$ einen Alkylrest mit bis zu 4 C-Atomen oder einen Phenyl- oder 4-Chlorphenylrest bedeutet,

$R^1$ einen Alkylrest mit bis zu 8 C-Atomen, einen Benzyl- 4-Chlorbenzyl-2,4-Dichlorbenzyl-, 4-Brombenzyl- oder 2-Phenylethylrest

und

$R^2$ einen Alkylrest mit bis zu 8 C-Atomen bedeutet.

2. Triazolyl-ketonoxime und -dioxime der Formel I gemäß Anspruch 1, in der

Ar einen Rest aus der Gruppe Biphenylyl, 1- und 2-Naphthyl, Phenyl, 2- und 4-Fluorphenyl, 3- und 4-Chlorphenyl, 4-Bromphenyl, 4-Methoxyphenyl, 4-Ethoxyphenyl, 4-Propoxyphenyl, 4-Methylphenyl, 2,4-Dimethylphenyl, 4-Ethylphenyl, 4-Propylphenyl, 4-n-Butylphenyl, 4-Isobutylphenyl, 4-tert.-Butylphenyl, 4-Pentylphenyl, 4-Phenoxyphenyl, 4-Nitrophenyl, 3- und 4-Trifluormethylphenyl, 4-Cyanphenyl, 4-Allylphenyl, 3,4-Dichlorphenyl, 2,4-Dichlorphenyl und 2,6-Dichlorphenyl,

n ganze Zahlen von 2 bis 6,

X Sauerstoff oder einen $=N-OR^3$-Rest, wobei

$R^3$ ein Wasserstoffatom oder einen Rest aus der Gruppe Methyl, Ethyl, 2-Methoxyethyl, n-Propyl, Isopropyl, n-Butyl, Isobutyl, Allyl, 2-Chlorallyl, 3-Chlorallyl, 2-Buten-1-yl, Pentenyl, Propargyl, Benzyl, 4-Fluorbenzyl, 4-Chlorbenzyl, 4-Trifluormethylbenzyl, 4-Nitrobenzyl, 4-Brombenzyl, 4-Methylbenzyl, 4-tert.-Butylbenzyl, 2,4-Dichlorbenzyl, 3,4-Dichlorbenzyl, 2,6-Dichlorbenzyl oder einen $CO-R^4$-Rest, wobei

$R^4$ Methyl, Chlormethyl, Brommethyl, Methoxymethyl, Ethyl, 2-Chlorethyl, n-Propyl, Isopropyl, Benzyl, Phenyl oder einen $-NH-R^5$-Rest, wobei

$R^5$ Methyl, Ethyl, Propyl, n-Butyl, Phenyl oder 4-Chlorphenyl,

$R^1$ Methyl, Ethyl, 2-Methoxyethyl, n-Propyl, Isopropyl, n-Butyl, Isobutyl, n-Pentyl, Isopentyl, n-Hexyl, n-Octyl, Benzyl, 4-Chlorbenzyl, 2,4-Dichlorbenzyl, 4-Brombenzyl, 2-Phenylethyl,

$R^2$ Methyl, Ethyl, n-Propyl, Isopropyl, n-Butyl, Isobutyl, tert.-Butyl, n-Pentyl oder n-Heptyl bedeuten.

3. Triazolyl-ketonoxim und -dioxim der Formel I gemäß Anspruch 1, in der

Ar Phenyl, 2- und 4-Fluorphenyl, 3- und 4-Chlorphenyl, 4-Bromphenyl, 4-Methylphenyl, 4-Ethylphenyl, 4-Propylphenyl, 4-tert.-Butylphenyl, 3- und 4-Trifluormethylphenyl, 3,4-Dichlorphenyl, 2,4-Dichlorphenyl und 2,6-Dichlorphenyl,

n die Zahl 2 oder 3,

X Sauerstoff oder einen $=N-OR^3$-Rest, wobei

$R^3$ ein Wasserstoffatom oder einen Rest aus der Gruppe Methyl, Ethyl, n-Propyl, n-Butyl, Allyl oder einen $CO-R^4$-Rest, wobei

$R^4$ Methyl, Ethyl, n-Propyl, Isopropyl, Phenyl, bedeuten.

4. Triazolyl-ketonoxime der Formel II

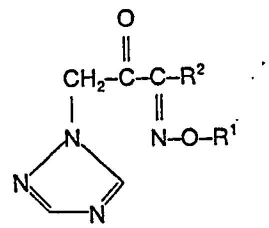

II,

in der $R^1$ und $R^2$ die im Anspruch 1, 2 oder 3 angegebenen Bedeutungen haben.

5. Die für Pflanzen verträglichen Salze und Metallkomplexe der Verbindungen I gemäß Anspruch 1, 2 oder 3.

6. Verfahren zur Herstellung von Triazolyl-ketonoximen oder -dioximen der Formel I gemäß Anspruch 1 oder 2, dadurch gekennzeichnet, daß man ein entsprechendes Ketonoxim der Formel II

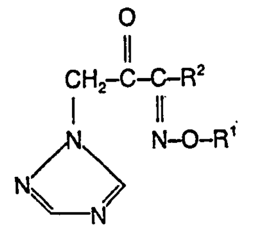

II,

oder dessen Alkalienolat mit einem entsprechenden Aryloxyalkylhalogenid der Formel III

$$Ar-O-(CH_2)_n-Y \qquad III,$$

in der Y Chlor, Brom oder Iod bedeutet, gegebenenfalls unter Zusatz eines Reaktionsbeschleunigers und/oder eines Phasentransferkatalysators bei einer ausreichenden Temperatur zwischen 0 und 100 °C umsetzt und die so erhaltenen Verbindungen in den Fällen, in denen X eine CO-Gruppe bedeutet, gegebenenfalls mit Hydroxylamin, Hydroxylamin-Derivaten der Formel $H_2N-O-R^3$ oder deren Salzen umsetzt.

7. Verfahren nach Anspruch 6, dadurch gekennzeichnet, daß man die Umsetzung des Ketonoxims II oder dessen Alkalienolat mit dem Aryloxyalkylhalogenid III in Gegenwart einer starken anorganischen Base vornimmt.

8. Verfahren zur Herstellung von Triazolyl-dioximen der Formel (I) gemäß Anspruch 1, in der X einen Rest $=N-O-R^3$ bedeutet, wobei $R^3$ von Wasserstoff verschieden ist, dadurch gekennzeichnet, daß man ein nach dem Verfahren des Anspruchs 6 erhaltenes Dioxim mit $R^3=H$ mit einem Alkylierungsmittel der Formel $R^3-Y$, in der $R^3$ – abgesehen von Wasserstoff – die in Anspruch 1 angegebene Bedeutung hat und Y Chlor, Brom und Iod bedeutet, oder mit einem Säurechlorid der Formel $R^4-COCl$, mit einem Säureanhydrid der Formel $(R^4-CO)_2O$, mit einem Isocyanat der Formel $R^5-N=C=O$ oder mit einem Carbamoylchlorid der Formel $R^5-NH-COCl$, in denen jeweils $R^4$ und $R^5$ die im Anspruch 1 angegebenen Bedeutungen haben, gegebenenfalls in Gegenwart eines Verdünnungsmittels und/oder einer anorganischen oder organischen Base und/oder eines Reaktionsbeschleunigers und/oder eines Phasentransferkatalysators bei einer ausreichenden Temperatur von bis zu 100 °C umsetzt.

9. Verfahren zur Herstellung von Triazolyl-dioximen der Formel I gemäß Anspruch 1, in der X einen Rest $=N-O-R^3$ bedeutet, dadurch gekennzeichnet, daß man ein Triazolyl-ketonoxim der Formel I gemäß Anspruch 1, in der X Sauerstoff bedeutet, mit einem Hydroxylamin-Derivat der Formel $H_2N-O-R^3$, in der $R^3$ die im Anspruch 1 angegebene Bedeutung hat, gegebenenfalls in Gegenwart eines basischen oder sauren Katalysators und gegebenenfalls in Gegenwart eines Verdünnungsmittels umsetzt.

10. Mittel zur Regulierung des Pflanzenwachstums, enthaltend eine oder mehrere Verbindungen gemäß Anspruch 1, 2 oder 3.

11. Mittel zur Regulierung des Pflanzenwachstums, enthaltend eine oder mehrere Verbindungen gemäß Anspruch 1, 2 oder 3 und einen flüssigen oder festen Trägerstoff sowie gegebenenfalls eine odere mehrere oberflächenaktive Mittel.

12. Verfahren zur Regulierung des Pflanzenwachstums, dadurch gekennzeichnet, daß man eine oder mehrere Verbindungen gemäß Anspruch 1, 2 oder 3 auf Pflanzen oder deren Lebensraum einwirken läßt.

13. Verfahren zur Herstellung von das Pflanzenwachstum regulierenden Mitteln, dadurch gekennzeichnet, daß man eine oder mehrere Verbindungen gemäß Anspruch 1, 2 oder 3 mit festen oder flüssigen Trägerstoffen sowie gegebenenfalls einem oder mehreren oberflächenaktiven Mitteln mischt.

**Claims**

1. A triazolylketone oxime or dioxime of the general formula I

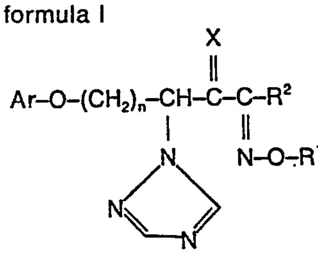

I,

where

Ar is aryl selected from the group consisting of biphenyl, naphthyl and phenyl, which may be substituted by halogen, nitrile, cyano or trifluoromethyl or by alkyl, alkoxy or alkenyl, each of up to 5 carbon atoms, and further by phenoxy,

n is an integer from 2 to 6,

X is oxygen or an $=N-OR^3$ radical, where $R^3$ is hydrogen, unsubstituted or halogen-substituted alkyl, alkenyl or alkynyl, each of up to 5 carbon atoms, or unsubstituted or halogen-, nitro-, cyano-, trifluoromethyl- or $C_1$–$C_4$-alkyl- or -alkoxy-substituted benzyl, or a $-CO-R^4$ radical, where $R^4$ is unsubstituted or halogen-substituted alkyl of up to 5 carbon atoms, phenyl or a $-NH-R^5$ radical, where $R^5$ is alkyl of up to 4 carbon atoms, phenyl or 4-chlorophenyl,

$R^1$ is alkyl of up to 8 carbon atoms, benzyl, 4-chlorobenzyl-2,4-dichlorobenzyl, 4-bromobenzyl or 2-phenylethyl, and

$R^2$ is alkyl of up to 8 carbon atoms.

2. A triazolylketone oxime or dioxime of the formula I as shown in claim 1, where

Ar is a radical selected from the group consisting of biphenylyl, 1- and 2-naphthyl, phenyl, 2- and 4-fluorophenyl, 3- and 4-chlorophenyl, 4-bromophenyl, 4-methoxyphenyl, 4-ethoxyphenyl, 4-propoxyphenyl, 4-methylphenyl, 2,4-dimethylphenyl, 4-ethylphenyl, m 4-propylphenyl, 4-n-butylphenyl, 4-isobutylphenyl, 4-tert.-butylphenyl, 4-pentylphenyl, 4-phenoxyphenyl, 4-nitrophenyl, 3- and 4-trifluoromethylphenyl, 4-cyanophenyl, 4-allylphenyl, 3,4-dichlorophenyl, 2,4-dichlorphenyl and 2,6-dichlorophenyl,

n is an integer from 2 to 6,

X is oxygen or an $=N-OR^3$ radical, where

$R^3$ is hydrogen or a radical selected from the group consisting of methyl, ethyl, 2-methoxyethyl, n-propyl, isopropyl, n-butyl, isobutyl, allyl, 2-chloroallyl, 3-chloroallyl, 2-buten-1-yl, pentenyl, propargyl, benzyl, 4-fluorobenzyl, 4-chlorobenzyl, 4-trifluoromethylbenzyl, 4-nitrobenzyl, 4-bromobenzyl, 4-methylbenzyl, 4-tert.-butylbenzyl, 2,4-dichlorobenzyl, 3,4-dichlorobenzyl, 2,6-dichlorobenzyl, or a $CO-R^4$ radical, where

$R^4$ ist methyl, chloromethyl, bromomethyl, methoxymethyl, ethyl, 2-chloroethyl, n-propyl, isopropyl, benzyl, phenyl or a $-NH-R^5$ radical, where

$R^5$ is methyl, ethyl, propyl, n-butyl, phenyl or 4-chlorophenyl,

$R^1$ is methyl, ethyl, 2-methoxyethyl, n-propyl, isopropyl, n-butyl, isobutyl, n-pentyl, isopentyl, n-hexyl, n-octyl, benzyl, 4-chlorobenzyl, 2,4-dichlorobenzyl, 4-bromobenzyl or 2-phenylethyl, and

$R^2$ is methyl, ethyl, n-propyl, isopropyl, n-butyl, isobutyl, tert.-butyl, n-pentyl or n-heptyl.

3. A triazolylketone oxime or dioxime of the formula I as shown in claim 1, where

Ar is phenyl, 2- and 4-fluorophenyl, 3- and 4-chlorophenyl, 4-bromophenyl, 4-methylphenyl, 4-ethylphenyl, 4-propylphenyl, 4-tert.-butylphenyl, 3- and 4-trifluoromethylphenyl, 3,4-dichlorophenyl, 2,4-dichlorophenyl and 2,6-dichlorophenyl,

n is 2 or 3,

X is oxygen or an $=N-OR^3$ radical, where

$R^3$ is hydrogen, a radical selected from the group consisting of methyl, ethyl, n-propyl, n-butyl and allyl, or a $CO-R^4$ radical, where

$R^4$ is methyl, ethyl, n-propyl, isopropyl of phenyl.

4. A triazolylketone oxime of the formula II

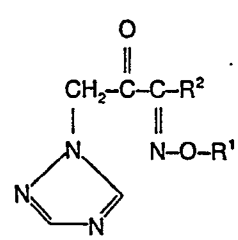

II,

where $R^1$ and $R^2$ are each as defined in claim 1, 2 or 3.

5. A plant-compatible salt or metal complex of a compound I as claimed in claim 1, 2 or 3.

6. A process for preparing a triazolylketone oxime or dioxime of the formula I as claimed in claim 1 or 2, which comprises reacting a corresponding ketone oxime of the formula II

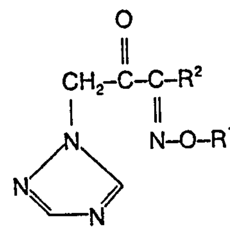

II,

or an alkali metal enolate thereof, with a corresponding aryloxyalkyl halide of the formula III

$$Ar-O-(CH_2)_n-Y \qquad III$$

where Y is chlorine, bromine or iodine, in the presence or absence of a reaction accelerant or phase transfer catalyst, at an adequate temperature of from 0 to 100 °C, and reacting the resulting compound if X is CO as desired with hydroxylamine, a hydroxylamine derivative of the formula $H_2N-O-R^3$ or a salt thereof.

7. A process as claimed in claim 6, wherein the reaction of ketone oxime II, or an alkali metal enolate thereof, with aryloxyalkyl halide III is carried out in the presence of a strong inorganic base.

8. A process for preparing a triazolylketone dioxime of the formula I as shown in claim 1, where X is an $=N-O-R^3$ radical and $R^3$ is different from hydrogen, which comprises reacting a dioxime where $R^3$ = H as obtained by the process of claim 6 with an alkylating agent of the formula $R^3-Y$, where $R^3$ is as defined in claim 1 but not hydrogen and Y is chlorine, bromine or iodine, with an acyl chloride of the formula $R^4-COCl$, with an anhydride of the formula $(R^4-CO)_2O$, with an isocyanate of the formula $R^5-N=C=O$ or with a carbamoyl chloride of the formula $R^5-NH-COCl$, in each of which $R^4$ and $R^5$ are each as defined in claim 1, in the presence or absence of a diluent or of an inorganic or organic base or of a reaction accelerant or of a phase transfer catalyst, at an adequate temperature of up to 100 °C.

9. A process for preparing a triazolylketone dioxime of the formula I as shown in claim 1, where X is an $=n-O-R^3$ radical, which comprises reacting a triazolylketone oxime of the formula I as shown in claim 1 where X is oxygen with a hydroxylamine derivative of the formula $H_2N-O-R^3$, where $R^3$ is as defined in claim 1, in the presence or absence of a basic or acidic catalyst and in the presence or absence of a diluent.

10. A plant growth regulator, containing one or more compounds as claimed in claim 1, 2 or 3.

11. A plant growth regulator, containing one or more compounds as claimed in claim 1, 2 or 3 and a liquid or solid carrier with or without one or more surfactants.

12. A method for regulating plant growth, which comprises treating plants or the habitat thereof with one or more compounds as claimed in claim 1, 2 or 3.

13. A process for preparing a plant growth regulator, which comprises mixing one or more compounds as claimed in claim 1, 2 or 3 with a solid or liquid carrier and with or without one or more surfactants.

**Revendications**

1. Triazolyl-cétone-oximes et dioximes de formule générale I

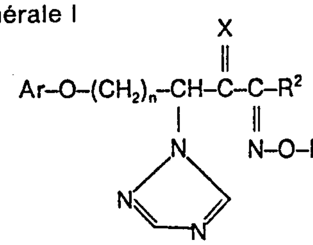

I,

dans laquelle

Ar représente un reste aryle du groupe comprenant les groupements biphényle, naphtyle ou phényle qui peuvent être substitués par des atomes d'halogène ou des groupements nitro, cyano ou trifluorométhyle ou par des groupements alkyle, alcoxy ou alcényle ayant chacun jusqu'à 5 atomes de carbone et en outre par un reste phénoxy,

n représente un nombre entier de 2 à 6,

X représente un atome d'oxygène ou un reste $=N-OR^3$, ou

$R^3$ représente un atome d'hydrogène, un reste alkyle, alcényle ou alcynyle éventuellement halogéno-substitué avec à chaque fois jusqu'à 5 atomes de carbone ou un reste benzyle éventuellement substitué par des atomes d'halogène ou par des groupements nitro, cyano, trifluorométhyle, alkyle ou alcocy ayant jusqu'à 4 atomes de carbone, ou un reste $-CO-R^4$ où $R^4$ représente un reste alkyle ayant jusqu'à 5 atomes de carbone éventuellement halogéno-substitué, un reste phényle ou un reste $-NH-R^5$, $R^5$ représentant un reste alkyle aynat jusqu'à 4 atomes de carbone ou un reste phényle ou 4-chlorophényle,

$R^1$ représente un reste alkyle ayant jusqu'à 8 atomes de carbone, un reste benzyle, 4-chlorobenzyle, 2,4-dichlorobenzyle, 4-bromobenzyle ou 2-phényléthyle, et

$R^2$ représente un reste alkyle ayant jusqu'à 8 atomes de carbone.

2. Triazolyl-cétone-oximes et dioximes de formule I selon la revendication 1, dans lesquelles

Ar représente un reste du groupe comprenant les groupements biphénylyle, 1- et 2-naphtyle, phényle, 2- et 4-fluorophényle, 3- et 4-chlorophényle, 4-bromophényle, 4-méthoxyphényle, 4-éthoxyphényle, 4-propoxyphényle, 4-méthylphényle, 2,4-diméthylphényle, 4-éthylphényle, 4-propylphényle, 4-n-butylphényle, 4-isobutylphényle, 4-tert.-butylphényle, 4-pentylphényle, 4-phénoxyphényle, 4-nitrophényle, 3- et 4-trifluorométhylphényle, 4-cyano-phényle, 4-allylphényle, 3,4-dichlorophényle, 2,4-dichlorophényle et 2,6-dichlorophényle,

n représente un nombre entier de 2 à 6,

X représente un atome d'oxygène ou un reste $=N-OR^3$, où

$R^3$ représente un atome d'hydrogène ou un reste du groupe comprenant les groupements méthyle, éthyle, 2-méthoxyéthyle, n-propyle, isopropyle, n-butyle, isobutyle, allyle, 2-chlorallyle, 3-chlorallyle, 2-butén-1-yle, pentényle, propargyle, benzyle, 4-fluorobenzyle, 4-chlorobenzyle, 4-trifluorométhylbenzyle, 4-nitrobenzyle, 4-bromobenzyle, 4-méthylbenzyle, 4-tert.-butylbenzyle, 2-4-dichlorobenzyle, 3,4-dichlorobenzyle, 2,6-dichlorobenzyle ou un reste $CO-R^4-$, où

$R^4$ représente un reste méthyle, chlorométhyle, bromométhyle, méthoxyméthyle, éthyle, 2-chloroéthyle, n-propyle, isopropyle, benzyle, phényle ou un reste $-N-R^5$,

$R^5$ représentant un groupement méthyle, éthyle, propyle, n-butyle, phényle ou 4-chlorophényle,

$R^1$ représente un groupement méthyle, éthyle, 2-méthoxyéthyle, n-propyle, isopropyle, n-butyle, isobutyle, n-pentyle, isopentyle, n-hexyle, n-octyle, benzyle, 4-chlorobenzyle, 2,4-dichlorobenzyle, 4-bromobenzyle, 2-phényléthyle,

$R^2$ représente un groupement méthyle, éthyle, n-propyle, isopropyle, n-butyle, isobutyle, tert.-butyle, n-pentyle ou n-heptyle.

3. Triazolyl-cétone-oximes ou dioximes de formule I selon la revendication 1, dans lesquelles

Ar représente un groupement phényle, 2- et 4-fluorophényle, 3- et 4-chlorophényle, 4-bromophényle, 4-méthylphényle, 4-éthylphényle, 4-propylphényle, 4-tert-butylphényle, 3- et 4-trifluorométhylphényle, 3,4-dichlorophényle, 2,4-dichlorophényle et 2,6-dichlorophényle,

n représente le nombre 2 ou 3,

X représente un atome d'oxygène ou un reste $=N-OR^3$, où

$R^3$ représente un atome d'hydrogène ou un reste du groupe comprenant les groupements méthyle, éthyle, n-propyle, n-butyle, allyle ou $CO-R^4$,

$R^4$ représentant un groupement méthyle, éthyle, n-propyle, isopropyle, phényle.

4. Triazolyl-cétone-oximes de formule II

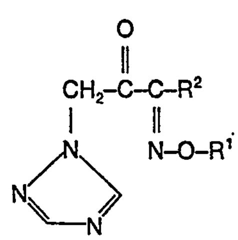

II,

dans laquelle $R^1$ et $R^2$ ont les définitions données dans les revendications 1, 2 ou 3.

5. Sels et complexes métalliques des composés I selon les revendications 1, 2 ou 3, compatibles avec les plantes.

6. Procédé de préparation de triazolyl-cétone-oximes ou dioximes de formule I selon les revendications 1 ou 2, caractérisé en ce qu'on fait réagir une cétone-oxime correspondante de formule II

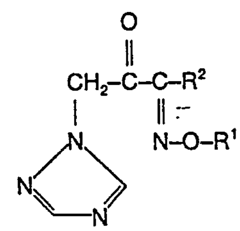

II,

ou un de ses énolates alcalins avec un halogénure d'aryloxyalkyle correspondant de formule III

$$Ar-O-(CH_2)_n-Y \qquad III,$$

dans laquelle Y représente un atome de chlore, de brome ou d'iode, éventuellement avec addition d'un accélérateur de réaction et/ou d'un catalyseur de transfert de phase à une température suffisante de 0 à 100 °C et on fait éventuellement réagir le composé ainsi obtenu dans le cas où X représente un groupe CO–, avec l'hydroxylamine, des dérivés d'hydroxylamine de formule $H_2N-O-R^3$ ou leurs sels.

7. Procédé selon la revendication 6, caractérisé en ce qu'on mène la réaction de la cétone-oxime II ou de son énolate alcalin avec l'halogénure d'aryloxyalkyle III en présence d'une base minérale forte.

8. Procédé de préparation de triazolyl-dioximes de formule I selon la revendication 1, dans laquelle X représente un reste $=N-O-R^3$, $R^3$ étant autre qu'un atome d'hydrogène, caractérisé en ce qu'on fait réagir une dioxime obtenue par le procédé selon la revendication 6 dans laquelle $R^3$ = H, avec un agent d'alkylation de formule $R^3-Y$, dans laquelle $R^3$ – à l'exception de l'hydrogène – a la signification donnée dans la revendication 1 et Y représente le chlore, le brome ou l'iode, ou avec un chlorure d'acide de formule $R^4-COCl$, avec un anhydride d'acide de formule $(R^4CO)_2O$, avec un isocyanate de formule $R^5-N=C=O$ ou avec un chlorure de carbamoyle de formule $R^5-NH-COCl$, où $R^4$ et $R^5$ ont les significations données dans la revendication 1, éventuellement en présence d'un diluant et/ou d'une base minérale ou organique et/ou d'un accélérateur de réaction et/ou d'un catalyseur de transfert de phase, à une température sufisante allant jusqu'à 100 °C.

9. Procédé de préparation de triazolyl-dioximes de formule I selon la revendication 1, dans laquelle X représente un reste $=N-O-R^3$, caractérisé en ce qu'on fait réagir une triazolyl-cétone-oxime de formule I selon la revendication 1 dans laquelle X représente l'oxygène, avec un dérivé d'hydroxylamine de formule $H_2N-O-R^3$ où $R^3$ a la signification donnée dans la revendication 1, éventuellement en présence d'un catalyseur acide ou basique et éventuellement en présence d'un diluant.

10. Agent de régulation de la croissance des plantes, contenant ou un plusieurs composés selon les revendications 1, 2 ou 3.

11. Agent de régulation de la croissance des plantes, contenant ou un plusieurs composés selon les revendications 1, 2 ou 3, et un support

liquide ou solide ainsi qu'éventuellement un ou plusieurs tensio-actifs.

12. Procédé de régulation de la croissance des plantes, caractérisé en ce qu'on fait agir un ou plusieurs composés selon les revendications 1, 2 ou 3 sur les plantes ou leur milieu de vie.

13. Procédé de préparation d'un agent de régulation de la croissance des plantes, caractérisé en ce qu'on mélange un ou plusieurs composés selon les revendications 1, 2 ou 3 avec un support liquide ou solide ainsi qu'éventuellement avec un ou plusieurs tensio-actifs.